# EUROPEAN PATENT SPECIFICATION

(11) **EP 2 785 356 B1**
(45) Date of publication and mention of the grant of the patent: **04.03.2020**
(21) Application number: 12852614.2
(22) Date of filing: 30.11.2012
(51) Int. Cl.: A61K 31/719, A61K 31/715, A61K 31/718, A61K 35/74, A61K 8/73, A61K 8/99, A61P 17/02, A61Q 19/00

(54) **USE OF PULLULAN FOR ALLEVIATING THE APPEARANCE OF SCARS OR SCAR TISSUE**
VERWENDUNG VOM PULLULAN ZUR VERMINDERUNG DER ERSCHEINUNG VON NARBEN ODER NARBENGEWEBE
UTILISATION DU PULLULANE POUR ATTÉNUER L'APPARENCE DES CICATRICES ET DU TISSU CICATRICIEL

(30) Priority: 01.12.2011 GB 201120724
(43) Date of publication of application: 08.10.2014
(73) Proprietor: Gauthier, Rene, Montreal, Quebec H1H 5V7 (CA); Marquis, Claude, Montreal, Quebec H1H 5V7 (CA)
(72) Inventor: Gauthier, Rene, Montreal, Quebec H1H 5V7 (CA); Marquis, Claude, Montreal, Quebec H1H 5V7 (CA)
(74) Representative: Hege, Frédéric
(86) International application number: PCT/CA2012/001110
(87) International publication number: WO 2013/078550

(56) References cited:
- CA-A1- 2 391 310
- CA-A1- 2 767 661
- US-A1- 2009 247 485
- US-A1- 2010 166 690
- US-A1- 2011 171 156
- US-A1- 2011 305 745
- US-B1- 6 329 343
- 'Scar Care Hypo, corrective cream-gel' SCAR CARE HYPO, CORRECTIVE CREAM-GEL 01 December 2010, XP055154817 Retrieved from the Internet: <URL:ittp://wwww.fpmarl:et.rulen/catalogue/ professional cosmetics/scar_care hypo> [retrieved on 2013-02-20]
- MORGANKOTH, P. ET AL.: 'Over-the-counter scar products for postsurgical patients: Disparities between online advertised benefits and evidence regarding efficacy' JOURNAL OF THE AMERICAN ACADEMY OF DERMATOLOGY vol. 61, 2009, ISSN 0190-9622 pages E31 - E47, XP026760859
- LOREN PICKART ET AL: "GHK Peptide as a Natural Modulator of Multiple Cellular Pathways in Skin Regeneration", BIOMED RESEARCH INTERNATIONAL, vol. 2015, 1 January 2015 (2015-01-01), pages 1-7, XP055471716, ISSN: 2314-6133, DOI: 10.1155/2015/648108
- Anonymous: "Mederma Advanced Scar Gel", , 2 May 2018 (2018-05-02), XP055471725, Retrieved from the Internet: URL:https://www.mederma.com/mederma-advanc ed-scar-gel/ [retrieved on 2018-05-02]
- Anonymous: "Revitol Scar Cream", , 30 April 2018 (2018-04-30), XP055471731, Retrieved from the Internet: URL:http://www.revitol.org.uk/revitol-scar -cream.html [retrieved on 2018-05-02]

## Description

### FIELD OF THE INVENTION

The present invention relates generally to skin care but more particularly to a system and method for alleviating the appearance of scars and/or scar tissue

### BACKGROUND OF THE INVENTION

Scars are not very desirable to most people. Scars can be the result of accidents or surgery, including reconstructive surgery such as for esthetic purposes. Scars or skin lesions can also be caused by a variety of treatments and or skin diseases such as burns, withdrawals of tattoos, laser treatments, dermabrasion, acids, acne cysts, hypertrophic scars, keloids, etc.

Although there have been several techniques, methods and technologies developed over the years to speed up skin regeneration and minimize the look of scars, such as silicone sheets placed over scars to cover a skin treatment compound as well as the skin itself, these techniques - such as silicone sheets -- can disintegrate and require help to keep them in place (medical adhesive tape, tailored gauze, etc.), and as such there is no effective system that is easy to implement and which gives excellent results. There is thus a need for improvement.

### SUMMARY OF THE INVENTION

In view of the foregoing disadvantages inherent in the known devices now present in the prior art, the present invention, which will be described subsequently in greater detail, is to provide objects and advantages which are:
To provide for an easy and reliable way for speeding up tissue repair and minimizing the appearance of scars.

In order to do so, the invention consists in combining two compounds, a first compound having properties that perform skin regeneration; and a second compound having properties that provide a physical barrier adapted to protect scar tissue by insulating it from ambient air while maintaining and protecting said first compound that is combined therewith.

The first compound is formed as a gel, and includes properties that is adapted to allow a user's skin to become more elastic, and the scar tissue to be more flexible. The second compound is formed as an occluding compound.

The occluding compound is an extracellular bacterial polysaccharide formed from starch.

The extracellular bacterial polysaccharide is PULLULAN, formed as a powder that is soluble in water and is adapted to form a film that can be applied as a layer over said first compound when applied to the skin of a user. PULLULAN includes the properties of high adhesion, lubrication, and film forming abilities.

A method of alleviating the appearance of scar tissue comprising the steps of:
a. providing a first compound having properties that perform skin regeneration;
b. applying and massaging the scar tissue with the first compound;
c. allowing the first compound to dry;
d. providing a second compound having properties that provide a physical barrier adapted to protect scar tissue by insulating it from ambient air while maintaining and protecting the first compound;
e. applying the second compound over the top of the first compound;
f. allowing the second compound to dry;
step c. is followed for at least five minutes;
step f. is followed for at least 8 minutes.
Steps d. through f. are followed at least once per day.

There has thus been outlined, rather broadly, the more important features of the invention in order that the detailed description thereof that follows may be better understood, and in order that the present contribution to the art may be better appreciated. There are additional features of the invention that will be described hereinafter and which will form the subject matter of the claims appended hereto.

In this respect, before explaining at least one embodiment of the invention in detail, it is to be understood that the invention is not limited in its application to the details of construction and to the arrangements of the components set forth in the following description or illustrated in the drawings. The invention is capable of other embodiments and of being practiced and carried out in various ways. Also, it is to be understood that the phraseology and terminology employed herein are for the purpose of description and should not be regarded as limiting.

As such, those skilled in the art will appreciate that the conception, upon which this disclosure is based, may readily be utilized as a basis for the designing of other structures, methods and systems for carrying out the several purposes of the present invention.

These together with other objects of the invention, along with the various features of novelty which characterize the invention, are pointed out with particularity in the claims annexed to and forming a part of this disclosure. For a better understanding of the invention, its operating advantages and the specific objects attained by its uses, reference should be made to the accompanying drawings and descriptive matter which contains illustrated preferred embodiments of the invention.

### BRIEF DESCRIPTION OF THE DRAWINGS

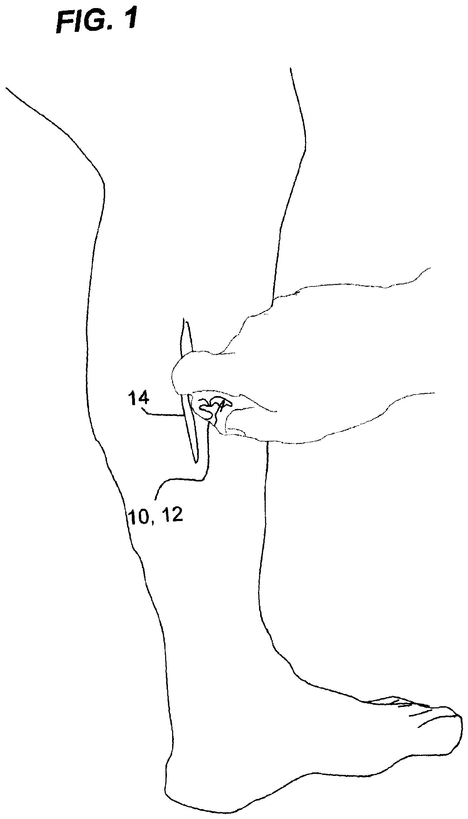

**Fig. 1** Schematic side view of the application of a first and or second compound on a scar.

### DETAILED DESCRIPTION OF THE PREFERRED EMBODIMENT

A system and method for alleviating the appearance of scars and/or scar tissue consists in the combination of two compounds:

A first compound (10) is a gel that has skin rejuvenation properties, which gives better elasticity to the skin, and allows the scar (14) to be more flexible. The use of this first compound or variants thereof is well known in the art of skin treatment and need not be further discussed herein.

A second compound (12) is an occluding substance, that offers protection from air (oxygen). It consists mainly of pullulan, a well known substance used in the food industry as a food additive in the manufacture of edible films used, for example, in breath freshener strips. Pullulan is an extracellular bacterial polysaccharide produced from starch. As an odorless white colored powder, pullulan is easily soluble in water to make clear and viscous solution. A pullulan film is an oxygen barrier that is 250 times stronger than HPMC film, and 9 time stronger than gelatin. Pullulan is more natural, involving no toxic chemicals. Pullulan is also much more inert than gelatin or HPMC, so there is no interaction with the products it is admixed with. It offers high adhesion, sticking, lubrication, and film forming abilities.

Using pullulan for the use described herein is new and unobvious since the repair of scars is totally unrelated to its use in the food industry.

Instead of using silicon sheets and/or silicon gel, using pullulan gives a superior protection by creating an excellent transparent protective thin film that is invisible and with remarkable adhesive properties. It is also better at creating an impermeable barrier to oxygen than current methods and has a flexible structure made of microfibers that can adapt to all types and shapes of scars. Pullulan is heat stable, biocompatible, non toxic, non-mutagenic, odorless, water soluble and is antistatic.

The two substances can easily be topically applied by the patient at home and requires twice a day applications (morning and evening) on and around the area to be treated.

The method of use on a user goes as follows:

In the morning, apply and gently massage the area with the first compound, that is the skin rejuvenation product. Drying time is about 5 minutes. If it takes more than 8 minutes to dry, this indicates that too much has been applied.

After the first compound has dried, makeup or sunscreen can be applied.

The area to be treated needs to have the first compound constantly for as long as required for full recovery.

Since the first compound is water soluble, washing will remove it completely, ready for the next application whether in the morning or in the evening.

Every evening, apply a thin layer of the first compound and wait a few minutes for it to dry, then apply a very small amount of the second compound over the layer of the first compound. Even if the latter is still a little damp, mixing the two products will not affect the results. It takes from 8 to 12 minutes for the two products to dry, depending on skin type. If it takes over 15 minutes, use a smaller dab of first compound and/or second compound. The second compound - pullulan -- must be applied once a day, in the evening only, for continuous occlusive skin repair overnight.

Because the second compound is distributed by way of a pump from a container, it is very clean and sterile. The second compound has exceptional adhesive properties, it is more comfortable and can easily cover all forms of scarring and this, regardless of its location on the body (elbow, knee, face, eyelids, etc.), something the silicone sheets cannot offer. This unobvious combinations, that is a first compound - known in the art of skin repair but heretofore used in combination with various

| | | | |
|---|---|---|---|
| **1** | Purified water (boled) | *Aqua* | 58.20 |
| **2** | Carbopol 940 | *Carbomer* | 1.00 |
| **3** | Purified water (boled) | *Aqua* | 25.00 |
| **4** | Pullulan ***MPC** | *Pullulan* | 5.00 |
| **5** | Terra-Pure Aloe Vera 200X | *Aloe Barbadensis Leaf Extract* | 0.10 |
| **6** | Zemea Propanediol | *Propanediol* | 5.00 |
| **7** | Glycerin 99,7 % USP | *Glycerin* | 1.00 |
| **8** | Allantoin | *Allantoin* | 0.50 |
| **9** | Velsan SC | *Sorbitan Caprylate* | 1.00 |
| **10** | Mikrokill ECT | *Benzyl Alcohol & Salicylic Acid & Glycerin & Sorb* | 1.00 |
| **11** | Chlorophyllin **sol. 2,5%** | *Chlorophyllin-Copper Complex* | 0.20 |
| **12** | Sodium Hydroxide, **sol. 30 %** | *Sodium Hydroxide* | 2.00 |
| **13** | Citric Acid, **sol. 50%** | *Citric Acid* | q.s. |

materials such as silicone sheets, etc - along with a second compound which has a new and unobvious use, makes this system and method unique.

As to a further discussion of the manner of usage and operation of the present invention, the same should be apparent from the above description. Accordingly, no further discussion relating to the manner of usage and operation will be provided.

The foregoing is considered as illustrative only of the principles of the invention. Further, since numerous modifications and changes will readily occur to those skilled in the art, it is not desired to limit the invention to the exact construction and operation shown and described.

## Claims

1. A set for alleviating the appearance of scar tissue, said system comprising two separate compounds; a first compound having properties that perform skin regeneration; and a second compound having properties that provide a physical barrier adapted to protect scar tissue by insulating it from ambient air while maintaining and protecting said first compound
**characterized in that**
said second compound is PULLULAN wehich is an extracellular bacterial polysaccharide; said PULLULAN is formed as a powder that is soluble in water and is adapted to form a film that can be applied as a layer over said first compound when applied to the skin of a user.

2. The set of claim 1, **characterized in that** said first compound is formed as a gel.

3. The set of claim 1 or 2, **characterized in that** said extracellular bacterial polysaccharide is formed from starch.

4. The set of one of claims 1 to 3, **characterized in that** said PULLULAN is formed as a powder that is soluble in water and is adapted to form a film that can be applied as a layer over said first compound when applied to the skin of a user.

5. Non-therapeutic method of alleviating the appearance of scar tissue, said method comprising the steps of:
a. providing a first compound having properties that perform skin regeneration;
b. applying and massaging said scar tissue with said first compound;
c. allowing said first compound to dry;
d. providing a second compound having properties that provide a physical barrier that protects scar tissue by insulating it from ambient air while maintaining and protecting said first compound; said second compound being formed as an occluding compound based on an extracellular bacterial polysaccharide, namely PULLULAN
e. applying said second compound over the top of said first compound;
fl allowing said second compound to dry.

6. The method of claim 5, **characterized in that** said PULLULAN is formed as a step c is followed for at least five minutes.

7. The method of claim 5 or 6, **characterized in that** step f. is followed for at least 8 minutes.

8. The method of one of claims 5 to 7, **characterized in that** steps d through f are followed at least once per day.

9. The method of one of claims 5 to 8, **characterized in that** said first compound is formed as a gel

10. The method of claim 5_characterized in that_use of PULLULAN for forming a physical barrier to protect scar tissue by insulating said scar tissue from ambient air.

## Patentansprüche

1. Ein Set zur Linderung des Erscheinungsbildes von Narbengewebe, wobei das System zwei getrennte Verbindungen umfasst; eine erste Verbindung mit Eigenschaften, die eine Hautregeneration durchführen; und eine zweite Verbindung mit Eigenschaften, die eine physikalische Barriere bereitstellen, die geeignet ist, Narbengewebe durch Isolierung von Umgebungsluft zu schützen, und die erste Verbindung zu erhalten und zu schützen,
**dadurch gekennzeichnet, dass**
die zweite Verbindung PULLULAN ist, das ein extrazelluläres bakterielles Polysaccharid ist; das PULLULAN wird als ein Pulver gebildet, das in Wasser löslich ist und so angepasst ist, dass es einen Film bildet, der als eine Schicht über die erste Verbindung aufgetragen werden kann, wenn es auf die Haut eines Benutzers aufgetragen wird.

2. Der Set nach Anspruch 1, **dadurch gekennzeichnet, dass** die erste Verbindung als ein Gel gebildet ist

3. Set nach Anspruch 1 oder 2, **dadurch gekennzeichnet, daß** das extrazelluläre bakterielle Polysaccharid aus Stärke gebildet wird.

4. Der Set nach einem der Ansprüche 1 bis 3, **dadurch gekennzeichnet, dass** das PULLULAN als ein Pulver gebildet ist, das in Wasser löslich ist und angepasst ist, um eine Folie zu bilden, die als eine Schicht über der ersten Verbindung aufgetragen werden kann, wenn sie auf die Haut eines Benutzers aufgetragen wird.

5. Nicht-therapeutisches Verfahren zur Linderung des Erscheinungsbildes von Narbengewebe, wobei das Verfahren die folgenden Schritte umfaßt:
a. Bereitstellen einer ersten Verbindung mit Eigenschaften, die eine Hautregeneration durchführen;
b. Auftragen und Massieren des Narbengewebes mit der ersten Verbindung;
c. Trocknenlassen der ersten Verbindung;
d. Bereitstellen einer zweiten Verbindung mit Eigenschaften, die eine physikalische Barriere bilden die das Narbengewebe schützt, indem sie es von der Umgebungsluft isoliert und gleichzeitig die erste Verbindung aufrechterhält und schützt; die zweite Verbindung wird als okkludierende Verbindung auf der Basis eines extrazellulären bakteriellen Polysaccharids, nämlich PULLULAN, gebildet;
e. Aufbringen der zweiten Verbindung über die erste Verbindung;
f. Trocknenlassen der zweiten Verbindung.

6. Verfahren nach Anspruch 5, **dadurch gekennzeichnet, dass** das PULLULAN aufgebracht wird nachdem Schritt c mindestens fünf Minuten lang befolgt wird.

7. Verfahren nach Anspruch 5 oder 6, **dadurch gekennzeichnet, dass** Schritt f. mindestens 8 Minuten lang befolgt wird.

8. Das Verfahren nach einem der Ansprüche 5 bis 7, **dadurch gekennzeichnet, dass** die Schritte d bis f mindestens einmal pro Tag befolgt werden.

9. Verfahren nach einem der Ansprüche 5 bis 8, **dadurch gekennzeichnet, dass** die erste Verbindung als Gel gebildet wird.

10. Verfahren nach Anspruch 5, **dadurch gekennzeichnet, dass** PULLULAN zur Bildung einer physikalischen Barriere zum Schutz von Narbengewebe durch Isolierung des Narbengewebes vor der Umgebungsluft verwendet wird.

## Revendications

1. Ensemble pour atténuer l'apparence d'un tissu cicatriciel, ledit ensemble comportant deux composés séparés ; un premier composé ayant des propriétés qui assurent la régénération de la peau ; et un second composé ayant des propriétés qui fournissent une barrière physique adaptée pour protéger le tissu cicatriciel en l'isolant de l'air ambiant tout en maintenant et en protégeant ledit premier composé
**caractérisé en ce que**
ledit second composé est le PULLULAN qui est un polysaccharide bactérien extracellulaire ; ledit PULLULAN étant formé d'une poudre qui est soluble dans l'eau et est adapté pour former un film qui peut être appliqué en couche sur ledit premier composé lorsqu'il est appliqué sur la peau d'un utilisateur.

2. Ensemble selon la revendication 1, **caractérisé en ce que** ledit composé est sous forme d'un gel.

3. Ensemble selon la revendication 1 ou 2, **caractérisé en ce que** ledit polysaccharide bactérien extracellulaire est formé à partir d'amidon.

4. Ensemble selon l'une des revendications 1 à 3, **caractérisé en ce que** ledit PULLULAN est formé d'une poudre soluble dans l'eau et adapté pour former un film qui peut être appliqué en couche sur ledit premier composé lorsqu'il est appliqué sur la peau d'un utilisateur.

5. Procédé non thérapeutique pour atténuer l'apparence d'un tissu cicatriciel, ledit procédé comportant les étapes suivantes :
a. fournir un premier composé ayant des propriétés qui assurent la régénération de la peau ;
b. appliquer et masser ledit tissu cicatriciel avec ledit premier composé ;
c. laisser sécher ledit premier composé ;
d. fournir un second composé ayant des propriétés qui fournissent une barrière physique qui protège le tissu cicatriciel en l'isolant de l'air ambiant tout en maintenant et en protégeant ledit premier composé ; ledit second composé étant formé comme un composé d'occlusion à base d'un polysaccharide bactérien extracellulaire, à savoir le PULLULAN ;
e. appliquer ledit deuxième composé par-dessus ledit premier composé ;
f. laisser sécher ledit second composé.

6. Procédé selon la revendication 5, **caractérisé en ce que** ledit PULLULAN est formé après que l'étape c ait été suivie pendant au moins cinq minutes.

7. Procédé selon la revendication 5 ou 6, **caractérisé en ce que** l'étape f. est suivie pendant au moins 8 minutes.

8. Procédé selon l'une des revendications 5 à 7, **caractérisée en ce que** les étapes d à f sont suivies au moins une fois par jour.

9. Procédé selon l'une des revendications 5 à 8, **caractérisé en ce que** ledit premier composé est sous forme d'un gel.

10. Procédé selon la revendication 5, **caractérisé en ce que** l'on utilise du PULLULAN pour former une barrière physique afin de protéger le tissu cicatriciel en isolant ledit tissu cicatriciel de l'air ambiant.
